# EUROPÄISCHE PATENTANMELDUNG

(11) **EP 1 880 684 A2**
(43) Veröffentlichungstag der Anmeldung: **23.01.2008**
(21) Anmeldenummer: 07021854.0
(22) Anmeldetag: 28.03.2002
(51) Int. Cl.: A61B 17/70

(54) **Vorrichtung zur Stabilisierung benachbarter Wirbelkörper umfassend ein Dämpfungselement**

(30) Priorität: 07.12.2001 WO PCT/CH01/00705
(62) Teilanmeldung aus: 02708113.2
(71) Anmelder: Synthes GmbH, 4436 Oberdorf (CH)
(72) Erfinder: Die Erfindernennung liegt noch nicht vor
(74) Vertreter: Lusuardi, Werther

(57) **Zusammenfassung**

Dämpfungselement (1) umfassend
A) zwei zu einer Längsachse (3) koaxiale oder parallele Federelemente (2;4) und axial endständig zwei Verbindungsteile (5;6); wobei
B) das erste Federelement (2) eine Federrate F aufweist;
C) das zweite Federelement (4) eine Federrate f aufweist; und
D) die Federraten F und f voneinander verschieden sind.

Vorrichtung zur Stabilisierung benachbarter Wirbelkörper mit
A) N Pedikelschrauben oder Pedikelhaken (12), wobei N ≥ 3 ist; und wobei
B) jede Pedikelschraube oder ein Pedikelhaken (12) Aufnahmemittel (13) umfasst, welche die Aufnahme von longitudinalen Befestigungselementen (7) gestattet, und wobei
C) ein als Feder wirkendes Element (14) zwischen zwei benachbarten Pedikelschrauben (12) oder Pedikelhaken eingesetzt ist.

## Beschreibung

Die Erfindung bezieht sich auf eine Dämpfungselement, gemäss dem Oberbegriff des Patentanspruchs 1, sowie auf eine Vorrichtung zur Stabilisierung benachbarter Wirbelkörper, gemäss dem Oberbegriff des Patentanspruchs 27.

Aus der FR-A-2 799 949 ist eine Wirbelsäulenfixations-Vorrichtung bekannt, welche aus einer Anzahl tulpenförmiger Pedikelschrauben besteht, welche statt des üblichen starren Längsträger mit einzelnen Spiralfederelementen untereinander verbunden sind. Die Länge der Spiralfedern ist zwar einstellbar, man erreicht aber damit nur eine Änderung der Federkraft zwischen zwei benachbarten Pedikelschrauben und damit zwischen zwei benachbarten Wirbel körpern. Ob die Federelemente in einem vorgespannten Zustand zwischen den Pedikelschrauben eingesetzt werden ist aus diesem Dokument nicht ersichtlich.

Aus der EP-A-0 516 567 ist eine weitere Wirbelsäulenfixations-Vorrichtung bekannt, welche aus einer Anzahl tulpenförmiger Pedikelschrauben besteht, welche statt des üblichen starren Längsträger mit einzelnen Dämpfungselementen untereinander verbunden sind. Nachteilig bei dieser Vorrichtung ist, dass nur Kompressionskräfte zwischen den Pedikelschrauben aufgenommen werden können. Da die Dämpfungselemente zudem eine fixe Länge besitzen, ist vorgesehen eine grössere Anzahl solcher Dämpfungselemente mit unterschiedlicher Länge bereitzustellen, um dann ein Dämpfungselement geeigneter Länge zwischen zwei implantierte Pedikelschrauben befestigen zu können. Dies ist umständlich und bedingt eine grössere Lagerhaltung an Dämpfungselementen verschiedener Längen.

Aus der EP-B-0 669 109 ist eine weitere Wirbelsäulenfixations-Vorrichtung bekannt, welche aus einer Anzahl von Pedikelschrauben mit durchbohrtem Kopf besteht, welche statt des üblichen starren Längsträgers mit einem durch die Bohrungen der Pedikelschrauben einziehbaren elastischen Kunststoffband untereinander verbunden sind. Zwischen den einzelnen Pedikelschrauben - auf dem Kunststoffband aufgereiht - sind hohlzylindrische Stützelemente, die allfällige Kompressionskräfte zwischen den Pedikelschrauben aufnehmen können. Die Nachteile dieser Vorrichtung sind vielfältig.

Erstens müssen das Kunststoffband und die Stützelemente - wie bei einer Perlenkette - in, bzw. zwischen die Bohrungen der bereits implantierten Pedikelschrauben eingefädelt werden, was für den Chirurgen umständlich und zeitraubend ist. Zweitens weist das - bis zu einem gewissen Grade elastische Kunststoffband - keine Vorspannung auf. Da die Länge der Stützkörpers auch bei dieser Vorrichtung fix ist, werden Sollbruchstellen am Stützkörper vorgeschlagen, so dass dieser intraoperativ an die effektive Distanz zwischen beiden betroffenen Pedikelschrauben vom Chirurgen zurechtgeschnitten werden kann. Dies ist für den Chirurgen umständlich und zeitraubend und dürfte in der Regel zu einem zu kurzen Stützelement führen, so dass seine Dämpfungswirkung erst mit einer gewissen Verzögerung eintritt, was natürlich unerwünscht ist.

Hier will die Erfindung Abhilfe schaffen. Der Erfindung liegt die Aufgabe zugrunde, ein kombiniertes, vorgespanntes Zug-Druck-Element zu schaffen, welches zwischen zwei Pedikelschrauben, bzw. Pedikelhaken befestigbar ist und einerseits auf Zug als Federelement mit einer gewissen Federrate und anderseits auf Druck als Dämpfungselement mit einer anderen Federrate wirkt.

Die Erfindung löst die gestellte Aufgabe mit einem Dämpfungselement, welches die Merkmale des Anspruchs 1 aufweist, sowie mit einer Vorrichtung zur Stabilisierung benachbarter Wirbelkörper, welche die Merkmale des Anspruchs 27 aufweist.

In der bevorzugten Ausführungsform des erfindungsgemässen Dämpfungselementes ist eines der Federelemente als Druckfeder angeordnet. Bei montiertem Dämpfungselement stehen die an den Enden der Federelemente angeordneten Verbindungsteile an den Enden des als Druckfeder angeordneten Federelementes an, so dass das erste Federelement auf Zug belastet werden kann und vorspannbar ist.

Die durch die Erfindung erreichten Vorteile sind im wesentlichen die folgenden:
- durch Wahl unterschiedlich langer Innenzylinder können die Dämpfungseigenschaften variiert werden.
- die bereits im vormontierten Zustand des Dämpfungselementes vorhandene Vorspannkraft ist klar definiert und kann dem Chirurgen in einer Auswahl zur Verfügung gestellt werden, welche den unterschiedlichen Körpergewichten der Patienten und den unterschiedlichen Indikationen entsprechen; und
- die Dämpfungselemente können nach dem Distrahieren der Wirbelkörper rasch und einfach zwischen die Pedikelschrauben eingelegt und daran fixiert werden.

In einer weiteren Ausführungsform des erfindungsgemässen Dämpfungselementes sind die beiden Verbindungsteile mit den Federelementen lösbar verbindbar. Die Verbindung zwischen den Verbindungsteilen und mindestens einem der Federelemente erfolgt vorzugsweise durch eine Gewindeverbindung. Damit ist erreichbar, dass je nach Anwendungsfall verschiedene, den Bedürfnissen angepasste Verbindungsteile eingesetzt werden können. Zudem bildet das erfindungsgemässe Dämpfungselement ein einheitliches Zug/Druck-Element , dessen Länge variabel einstellbar ist.

In einer anderen Ausführungsform des erfindungsgemässen Dämpfungselementes ist mindestens eines der beiden Verbindungsteile einstückig mit mindestens einem der Federelemente ausgeführt. Durch eine solche Ausgestaltung ist eine kompaktere Bauweise des erfindungsgemässen Dämpfungselementes erreichbar.

Die Verbindungsteile können je nach Anwendung des erfindungsgemässen Dämpfungselementes stabförmig oder als Büchse ausgebildet sein, wobei je ein Verbindungsteil zur Längsachse koaxial an einem Ende der Federelemente angeordnet ist. Die Büchse weist vorzugsweise eine zur Längsachse koaxiale Zentralbohrung auf und umfasst Arretiermittel zur Fixierung eines in der Zentralbohrung eingeführten Stabes. Durch diese Ausgestaltung des Dämpfungselementes ist erreichbar, dass koaxial zur Längsachse ein stabförmiges Befestigungselement oder ein stabförmiges Verbindungsteil eines weiteren Dämpfungselementes mit dem als Büchse ausgestalteten Verbindungsteil des ersten Dämpfungselementes verbindbar ist.

Vorzugsweise umfassen die Arretiermittel mindestens eine Schraube, welche quer zur Längsachse in die Büchse einschraubbar ist und zur Fixierung eines in die Zentralbohrung der Büchse eingeführten stabförmigen Teiles dient. Durch Verwendung einer radial nicht über die Büchse hinausragende Stiftschraube als Arretiermittel ist eine kompakte Bauweise des Dämpfungselementes erreichbar, was für die Anwendung des Dämpfungselementes innerhalb einer Vorrichtung zur Stabilisierung von Wirbelkörpern vorteilhaft ist.

Die Verbindungsteile an den beiden Enden der Federelemente können je nach Anwendung des Dämpfungselementes beide stabförmig, beide als Büchsen oder eines als Büchse und das Andere stabförmig ausgestaltet sein.

Bei einer Anwendung eines oder mehrerer erfindungsgemässen Dämpfungselemente innerhalb einer Vorrichtung zur Stabilisierung von benachbarten Wirbelkörpern wird vorzugsweise eines der Verbindungselemente an einer Pedikelschraube oder einem Pedikelhaken fixiert und durch das zweite Verbindungselement mit einem stabförmigen Längsträger verbunden. Dazu eignet sich die Ausgestaltung der Verbindungselemente derart, dass eines der Verbindungselemente als Büchse ausgeführt ist während das zweite Verbindungsteil stabförmig ausgebildet ist. Der Längsträger wird in die Zentralbohrung der Büchse eingeführt und mittels der Arretiermittel in der Büchse fixiert, während das stabförmige Verbindungsteil in die üblicherweise als Kanal ausgestalteten Aufnahmemittel an den Pedikelschrauben oder Pedikelhaken einführbar und dort ebenfalls fixierbar ist. Vorzugsweise weisen die stabförmig ausgestalteten Verbindungsmittel axial eine Länge auf, welche grösser als die Länge der Aufnahmemittel ist, so dass das Dämpfungselement je nach bedarf axial verschoben und justiert werden kann.

Zur Vereinfachung einer kombinierten Anwendung von stabförmigen Befestigungselementen, beispielsweise Längsträgern und erfindungsgemässen Dämpfungselementen innerhalb einer Vorrichtung zur Stabilisierung von benachbarten Wirbelkörpern werden der Durchmesser D der Zentralbohrung des als Büchse ausgestalteten Verbindungsteiles und der Durchmesser d des stabförmigen Verbindungsteiles so gewählt, dass D ≅ d ist.

In einer anderen Ausführungsform des erfindungsgemässen Dämpfungselementes sind die Federelemente so ausgestaltet, dass sie eine konstante Federrate aufweisen. Damit ist erreichbar, dass bei Entlastung des Dämpfungselementes der Zustand der unbelasteten Federelemente wieder herstellbar ist.

In einer anderen Ausführungsform des erfindungsgemässen Dämpfungselementes weist dieses einen zur Längsachse orthogonalen, nierenförmigen Querschnitt auf. Die Vorteile einer solchen Ausführung liegen darin, dass bei der Implantation eines oder mehrerer Dämpfungselemente, beispielsweise innerhalb einer Wirbelsäulenfixation, diese mit Berücksichtigung von Wirbelfortsätzen oder anderen Implantatteilen günstiger plazierbar sind.

In einer weiteren Ausführungsform des erfindungsgemässen Dämpfungselementes ist das zweite Federelement im Innern des ersten Federelementes angeordnet. Vorzugsweise ist das zweite Federelement als Druckfeder ausgebildet, so dass bei zusammengebautem Dämpfungselement eine Vorspannung mindestens des ersten Federelementes erreichbar ist. Ferner ist die Anordnung der beiden Federelemente vorzugsweise konzentrisch. Der Vorteil dieser Ausführungsform liegt ebenfalls in den damit erreichbaren geringen Aussenabmessungen des Dämpfungselementes.

In wiederum einer weiteren Ausführungsform des erfindungsgemässen Dämpfungselementes weist das im Innern des ersten Federelementes angeordnete und als Druckfeder ausgestaltete zweite Federelement eine zur Längsachse koaxiale Zentralbohrung auf, deren Durchmesser vorzugsweise dem Durchmesser der Zentralbohrung in der Büchse entspricht. Dadurch ist eine längere axiale Führung eines in die Zentralbohrungen eingeführten stabförmigen Teiles möglich, wodurch eine höhere Stabilität der Verbindung zwischen dem Dämpfungselement und dem stabförmigen Teil erreichbar ist.

In einer anderen Ausführungsform des erfindungsgemässen Dämpfungselementes ist das erste Federelement als Schraubenfeder ausgestaltet. Der schraubenförmige Schlitz der Schraubenfeder wird vorzugsweise durch eine komplementär schraubenförmige und elastisch deformierbare Erhebung am zweiten Federelement verschlossen. Die Federwendel der Schraubenfeder kann dabei auch mehrgängig ausgestaltet sein. Vorzugsweise ist das zweite Federelement aus einem giessfähigen Material hergestellt, so dass das zweite Federelement in das bereits fertiggestellte erste Federelement eingiessbar ist.

In einer Ausführungsform des erfindungsgemässen Dämpfungselementes ist das als Druckfeder ausgestaltete Federelement aus einem Polymer, vorzugsweise aus Polycarbonurethan (PCU) hergestellt.

In einer weiteren Ausführungsform des erfindungsgemässen Dämpfungselementes ist das als Büchse ausgestaltete Verbindungsteil mit dem Federelement einstückig ausgebildet, wodurch eine einfache Bauweise des Dämpfungselementes erreichbar ist. Dabei sind auch Ausgestaltungen des Dämpfungselementes möglich, wo beide Verbindungsteile mit dem ersten Federelement einstückig ausgebildet sind.

In verschiedenen Ausführungsformen des erfindungsgemässen Dämpfungselementes sind die Federkonstante F des ersten Federelementes und die Federkonstante f des zweiten Federelementes voneinander verschieden. Die beiden Federkonstanten F;f können sich dabei um einen Faktor von mindestens 1,2, vorzugsweise mindestens 5 unterscheiden. Dadurch ist der Vorteil erreichbar, dass die Federkräfte des Dämpfungselementes bei Belastung auf Zug oder Druck voneinander verschieden sind. Je nach Anwendungsbereich des Dämpfungselementes können sich die Federkonstanten auch um einen Faktor zwischen 10 und 100 unterscheiden.

In weiteren Ausführungsformen des erfindungsgemässen Dämpfungselementes beträgt die Federrate f des zweiten Dämpfungselementes zwischen 50 N/mm und 5000 N/mm, vorzugsweise zwischen 100 N/mm und 2000 N/mm.

Weitere vorteilhafte Ausgestaltungen der Erfindung sind in den abhängigen Ansprüchen gekennzeichnet.

Die erfindungsgemässe Vorrichtung zur Stabilisierung benachbarter Wirbelkörper umfasst im wesentlichen mehrere, mit verschiedenen Befestigungsmitteln verbindbare Pedikelschrauben oder Pedikelhaken. Zwischen zwei Pedikelschrauben oder Pedikelhaken können als Befestigungsmittel beispielsweise stabförmige Längsträger, Federn oder erfindungsgemässe Dämpfungselemente eingesetzt werden.

In der bevorzugten Ausführungsform der erfindungsgemässen Vorrichtung umfassen die Pedikelschrauben oder Pedikelhaken Aufnahmemittel, welche die axial verschiebbare Aufnahme der Befestigungselemente, beispielsweise von stabförmigen Längsträgern, respektive der stabförmig ausgebildeten Verbindungsteile an den erfindungsgemässen Dämpfungselementen gestatten. Zur Fixierung der Befestigungselemente in den Aufnahmemitteln umfassen die Pedikelschrauben oder Pedikelhaken Feststellmittel, welche beispielsweise endständig angeordnet sein können und aus Klemmschrauben oder Klemmuttern bestehen können. Die erfindungsgemässen Vorrichtung ist durch den Einsatz von einerseits rigiden Elementen, wie beispielsweise Längsträger und andererseits dämpfenden Elementen bezüglich ihrer Stabilität an die jeweiligen Bedingungen anpassbar.

In einer anderen Ausführungsform der erfindungsgemässen Vorrichtung umfasst mindestens eine Pedikelschraube oder ein Pedikelhaken Aufnahmemittel, welche zugleich die Aufnahme von zwei parallelen longitudinalen Befestigungselementen gestattet. Auf diese Weise wird ermöglicht, dass ein als Feder wirkendes Element, beispielsweise ein erfindungsgemässes Dämpfungselement zur Befestigung zwischen mindestens einer mit Aufnahmemitteln versehenen Pedikelschraube oder Pedikelhaken und einer weiteren, benachbarten Pedikelschraube oder Pedikelhaken einsetzbar ist.

Pedikelschrauben oder Pedikelhaken mit Aufnahmemitteln, welche gestatten, dass zugleich zwei parallele, longitudinale Befestigungselemente mit der Pedikelschraube oder dem Pedikelhaken verbindbar sind, sind beispielsweise aus der US-A-4 653 481 HOWLAND bekannt. Die erfindungsgemässen Dämpfungselemente können analog zu den im vorangehend erwähnten Patent gezeigten Längsträgern mittels an den Verbindungsteilen parallel zur Längsachse angebrachten Stäben, beispielsweise in parallelen Kanälen an den Schraubenköpfen fixiert werden. Die durch eine solche Anordnung mögliche Verschiebbarkeit des Dämpfungselementes parallel zur Längsachse in den Kanälen gestattet, ein vor der Implantation auf eine gewünschte Federkraft vorgespanntes, erfindungsgemässes Dämpfungselement ohne weitere Manipulation am Dämpfungselement in die Aufnahmemittel an den Pedikelschrauben einzufügen. Die Längenkompensation bei verschiedenen Abständen zwischen benachbarten Pedikelschrauben oder Pedikelhaken erfolgt über die axiale Verschiebbarkeit der endständig parallel zur Längsachse angeordneten, stabförmig ausgebildeten Verbindungselemente an den erfindungsgemässen Dämpfungselemente in den ebenfalls zur Längsachse parallelen Kanälen.

Die Vorspannung des Dämpfungselementes gestattet beispielsweise die Berücksichtigung verschiedener Instabilitäten, Indikation oder des Gewichtes des Patienten. Das Dämpfungselement ist bei einer Extension der damit verbundenen Wirbelsäulenteile auf Druck belastet, während es bei einer Flexion der damit verbundenen Wirbelsäulenteile auf Zug belastet wird. Die Wahl des Federmateriales, beispielsweise ein Polymer, vorzugsweise Polycarbonaturethan (PCU) für das auf Druck belastete Federelement und ein Metall für das auch auf Zug belastete Federelement, die Wahl der geometrischen Abmessungen sowie die eingestellte Vorspannung des auch auf Zug belasteten Federelementes gestatten eine optimale Anpassung der erfindungsgemässen Vorrichtung an die biomechanischen Gegebenheiten bei einem Patienten.

Die Vorteile der erfindungsgemässen Vorrichtung sind im wesentlichen die folgenden:
- harmonischer Steifigkeitsübergang vom stabiliserten Wirbelsäulensegment zu den gesunden Wirbelsäulensegementen.
- Die Dämpfungselemente können wahlweise mit rigiden Stäben segementweise kombiniert werden.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Längsschnitt durch eine Ausführungsform des erfindungsgemässen Dämpfungselementes;
Fig. 2 eine Ansicht einer Ausführungsform der erfindungsgemässen Vorrichtung zur Stabilisierung benachbarter Wirbelkörper;
Fig. 3 eine Explosionsdarstellung einer Ausführungsform des erfindungsgemässen Dämpfungselementes;
Fig. 4 eine Ansicht einer Ausführungsform des erfindungsgemässen Dämpfungselementes;
Fig. 5 eine perspektivische Darstellung einer Ausführungsform des erfindungsgemässen Dämpfungselementes;
Fig. 6 eine Aufsicht auf eine Ausführungsform des erfindungsgemässen Dämpfungselementes;
Fig. 7 einen Längsschnitt durch eine Ausführungsform des erfindungsgemässen Dämpfungselementes;
Fig. 8 eine Ansicht einer Ausführungsform der erfindungsgemässen Vorrichtung bei ihrer Anwendung zur Stabilisierung von benachbarten Wirbelkörpern; und
Fig. 9 eine perspektivische Ansicht einer Ausführungsform der erfindungsgemässen Vorrichtung.

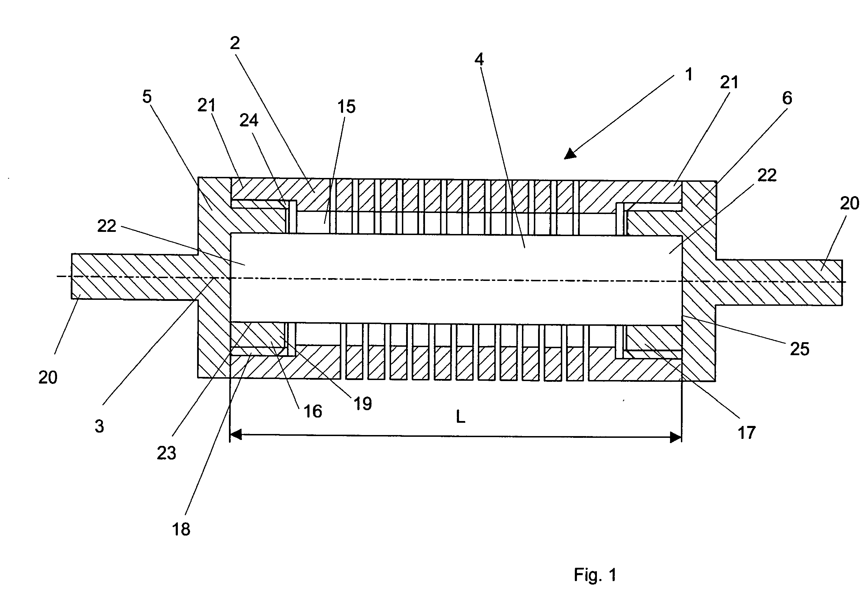

Die Fig. 1 zeigt eine Ausführungsform des erfindungsgemässen Dämpfungselementes 1 mit zwei zur Längsachse 3 konzentrisch angeordneten Federelementen 2;4. Das erste Federelement 2 ist als Schraubenfeder mit einem zentralen Hohlraum 15 ausgeführt, während das zweite Federelement 4 stabförmig ausgestaltet ist und im Hohlraum 15 angeordnet ist. Die endständigen Verbindungsteile 5;6 sind ebenfalls zur Längsachse 3 koaxial angeordnet und weisen gegen die Federelemente 2;4 gerichtet, je ein zur Längsachse 3 koaxiales Gewindestück 16;17 mit einem Aussengewinde 18 auf. Das erste Federelement 2 ist an seinen axialen Enden 21 mit im Hohlraum 15 angebrachten Innengewinden 24 versehen, welche zu den Aussengewinden 18 komplementär ausgestaltet sind, so dass die Gewindestücke der Verbindungsteile 5;6 in das erste Federelement 2 einschraubbar sind. Ferner umfasst jedes Verbindungsteil 5;6 eine zur Längsachse 3 koaxial angeordnete und am inneren Ende 19 des Verbindungsteils 5;6 offene Vertiefung 23, so dass das stabförmig ausgebildete zweite Federelement 4 an seinen axialen Enden 22 in den Vertiefungen 23 aufnehmbar ist. Ferner sind die Verbindungsteile 5;6 an ihren äusseren Ende 20 koaxial stabförmig ausgebildet. Bei montiertem Dämpfungselement 1 liegen die Enden 22 der zweiten Feder 4 an den zur Längsachse 3 orthogonalen Stirnflächen 25 der Vertiefungen 23 auf, so dass die Verbindungsteile 5;6 zwischen diesen Stirnflächen 25 einen Abstand L aufweisen. Dieser Abstand L sowie die Länge des nicht deformierten ersten Federelementes 2 sind so bemessen, dass beim Einschrauben der Gewindestücke 16;17 in die Innengewinde 24 das erste Federelement 2 axial um eine gewünschte Länge gedehnt wird, wodurch das Dämpfungselement 1 eine Vorspannung erhält.

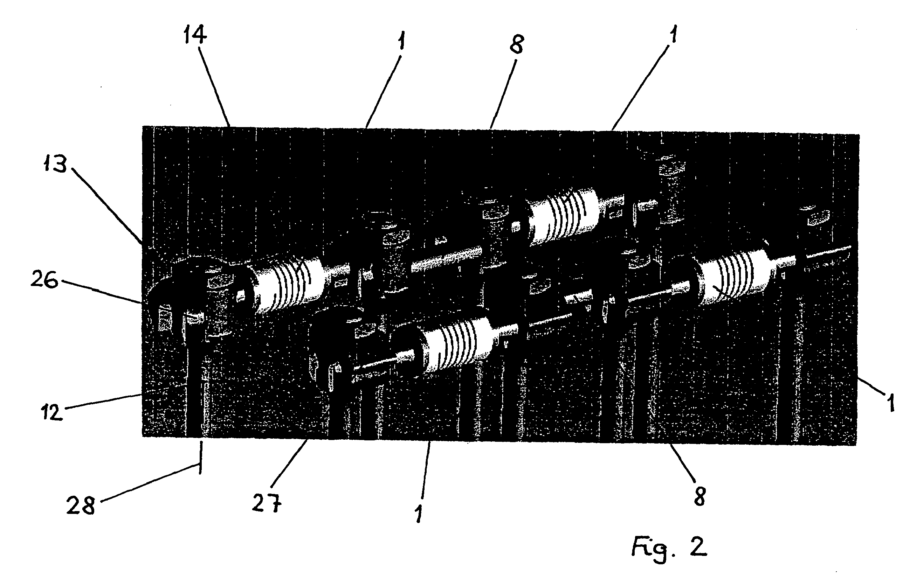

In Fig. 2 ist eine Ausführungsform der erfindungsgemässen Vorrichtung am Beispiel einer Vorrichtung zur Stabilisierung benachbarter Wirbelkörper (nicht gezeichnet) dargestellt. Mehrere Pedikelschrauben oder -haken 12 sind so an den Pedikeln der zu verbindenden Wirbelkörper befestigt, dass ihre Zentralachsen 28 quer zur Wirbelsäulenlängsachse angeordnet sind. Die Aufnahmemittel 13 an den Pedikelschrauben oder -haken 12 sind zu den Zentralachsen 28 senkrecht angeordnet und als Kanäle 26 ausgebildet. In diesen Kanälen 26 sind die stabförmigen, äusseren Enden 20 der Verbindungsteile 5;6 (Fig. 1) einführbar, so dass die Dämpfungselemente 1 in den Kanälen 26 axial verschiebbar sind, bevor sie mittels Schrauben 27 relativ zu den Pedikelschrauben oder -haken 12 fixiert werden. Die Aufnahmemittel 13 an den Pedikelschrauben oder -haken 12 umfassen je zwei parallele Kanäle 26, so dass an einer Pedikelschraube oder -haken 12 neben einem Dämpfungselement 1 beispielsweise ein stabförmiges Befestigungselement 7 fixierbar ist.

Fig. 3 zeigt eine Ausführungsform des erfindungsgemässen Dämpfungselementes 1 mit einem als Schraubenfeder ausgestalteten, ersten Federelement 2, einem stabförmig ausgebildeten, zweiten Federelement 4 und zwei zur Längsachse 3 koaxial angeordneten Verbindungsteilen 5;6.

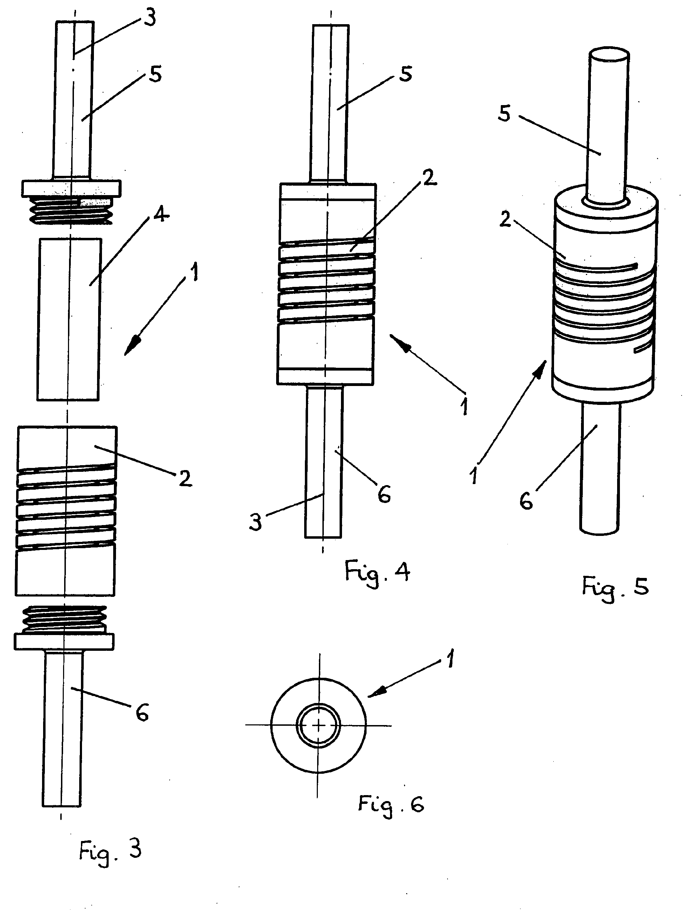

Fig. 4 und 5 zeigen eine Ausführungsform des erfindungsgemässen Dämpfungselementes 1 mit einem als Schraubenfeder ausgestalteten ersten Federelement 2 und zwei zur Längsachse 3 koaxial mit dem ersten Federelement 2 verbundenen Verbindungsteilen 5;6.

In Fig. 6 ist eine Ausführungsform des erfindungsgemässen Dämpfungselementes 1 dargestellt, welches einen zur Längsachse 3 orthogonalen, kreisförmigen Querschnitt aufweist. Andere Querschnittsformen, beispielsweise ovale oder elliptische Querschnitte, welche die Implantation des Dämpfungselementes 1 begünstigen sind ebenfalls möglich.

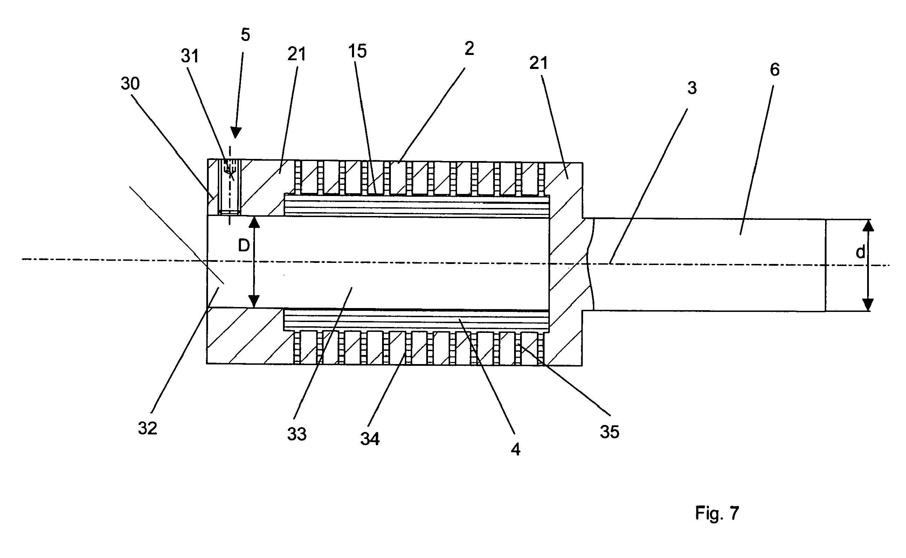

In Fig. 7 ist eine Ausführungsform des erfindungsgemässen Dämpfungselementes 1 dargestellt, welches eine zur Längsachse 3 koaxial angeordnete, kreiszylindrische Schraubenfeder als erstes Federelement 2 umfasst. Dieses erste Federelement 2 weist einen schraubenlinienförmigen Schlitz 34 auf und ist mit einem zur Längsachse 3 koaxialen, nur an einem Ende 21 des ersten Federelementes 2 offenen Hohlraum 15 ausgestattet. An jedem der beiden axialen Enden 21 ist ein Verbindungsteil 5;6 angeordnet, wobei die beiden Verbindungsteile 5;6 in dieser Ausführungsform mit dem ersten Federelement 2 einstückig sind. Das erste Verbindungsteil 5 ist als Büchse 30 mit einer zur Längsachse 3 koaxialen Zentralbohrung 32 ausgebildet, während das zweite Verbindungsteil 6 als ebenfalls zur Längsachse 3 koaxialer Stab ausgebildet ist. Das zweite, stabförmige Verbindungsteil 6 ist an dem Ende 21 des ersten Federelementes 2 angeordnet, wo der Hohlraum 15 axial geschlossen ist. Die Zentralbohrung 32 im ersten Verbindungsteil 5 weist einen Durchmesser D auf. Das zweite Verbindungsteil 6 ist ebenfalls kreiszylindrisch ausgestaltet und weist einen Durchmesser d auf, welcher in der hier dargestellten Ausführungsform des Dämpfungselementes 1 gleich gross wie der Durchmesser D der Zentralbohrung 32 im ersten Verbindungsteil 5 ist. Somit lässt sich beispielsweise ein zu einer Vorrichtung zur Stabilisierung von Wirbelkörpern gehörender Längsstab oder das zweite, stabförmige Verbindungsteil 6 eines weiteren Dämpfungselementes 1 in die Zentralbohrung 32 einführen. Mittels Arretiermitteln 31, welche in der hier dargestellten Ausführungsform des Dämpfungselementes 1 als quer zur Längsachse 3 in die Büchse 30 einschraubbare Stiftschraube ausgebildet sind, ist ein solcher in die Zentralbohrung 32 eingeführter Stab fixierbar. Das zweite Federelement 4 ist koaxial zur Längsachse 3 im Hohlraum 15 des ersten Federelementes 2 angeordnet und weist eine zum schraubenlinienartigen Schlitz 34 im ersten Federelement 2 komplementäre schraubenlinienartige, elastisch deformierbare Erhebung 35 auf, welche den Schlitz 34 im ersten Federelement 2 verschliesst. Ferner ist das zweite Federelement 4 mit einer zur Längsachse 3 koaxialen Zentralbohrung 33 ausgestattet, welche denselben Durchmesser aufweist wie die Zentralbohrung 32 in der Büchse 30.

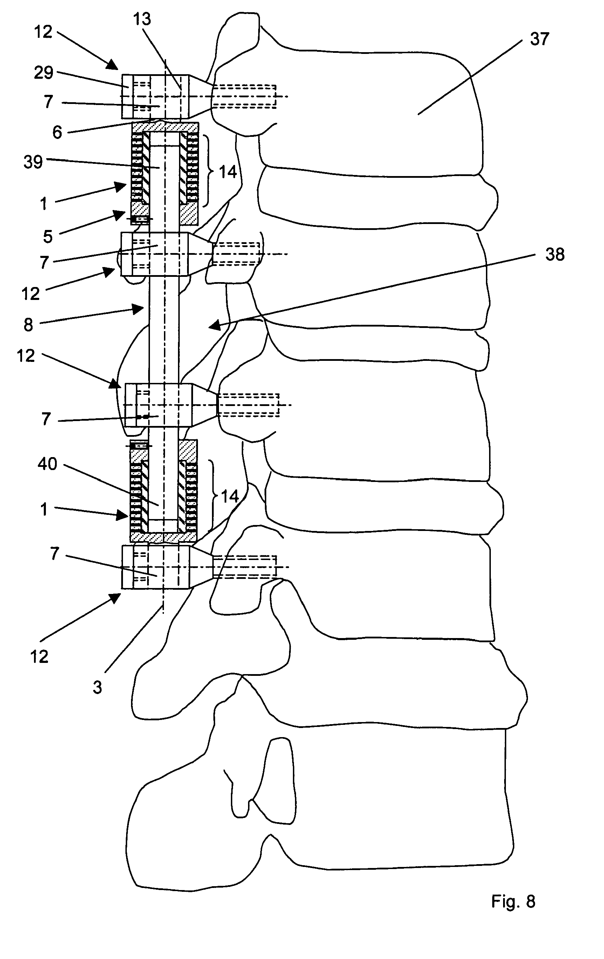

Fig. 8 zeigt eine Ausführungsform der erfindungsgemässen Vorrichtung 38 in einer Anwendung zur Stabilisierung von benachbarten Wirbelkörpern 37. In vier aneinandergrenzenden Wirbelkörpern 37 ist je eine Pedikelschraube 12 eingeschraubt. Die Vorrichtung 38 umfasst koaxial zur Längsachse 3 angeordnet jeweils zwischen einem der äusseren der vier aneinandergrenzenden Wirbelkörper 37 und dem benachbarten inneren Wirbelkörper 37 ein als Feder wirkendes Element 14 sowie zwischen den beiden benachbarten inneren Wirbelkörpern 37 einen stabförmigen Längsträger 8. Die als Feder wirkenden Elemente 14 bestehen aus Dämpfungselementen 1, welche je an einem ihrer Enden 21 (Fig. 7) ein stabförmiges Verbindungsteil 6 und am anderen Ende 21 eine Büchse 30 (Fig. 7) umfassen. Die stabförmigen Verbindungsteile 6 dienen als Befestigungselemente 7, welche in die Aufnahmemittel 13 der beiden bezüglich der Vorrichtung 38 endständigen Pedikelschrauben 12 eingeführt sind und dort mittels der als Schrauben ausgeführten Feststellmittel 29 mit den Pedikelschrauben lösbar verbunden sind. Die als Büchsen 30 (Fig. 7) ausgeführten Verbindungsteile 5 der Dämpfungselemente 1 sind gegen die inneren beiden der vier zu stabilisierenden Wirbelkörper 37 gerichtet. Zwischen den beiden Dämpfungselementen 1 ist ein stabförmiger Längsträger 8 eingesetzt, wobei der Längsträger 8 so ausgebildet ist, dass die Befestigungselemente 7 in den Aufnahmemitteln 13 der beiden inneren Pedikelschrauben 12 durch axiale Segmente des Längsträgers 8 gebildet werden. Der Längsträger 8 ist einerseits mittels der als Schrauben ausgebildeten Feststellmittel 29 der beiden inneren Pedikelschrauben 12 mit der Vorrichtung 38 verbunden und andererseits mit seinen beiden Enden 39;40 in die Zentralbohrungen 32;33 in den Verbindungsteilen 5 und den zweiten Federelementen 4 (Fig. 7) eingeführt und dort mittels der Arretiermittel 31 (Fig. 7) lösbar blockiert. Je nach Anwendungsfall sind auch andere zur Längsachse 3 koaxiale Kombinationen von Längsträger 8 und Dämpfungselementen 1 möglich. Anstelle eines Längsträgers 8 können mehrere Längsträger 8 eingesetzt werden oder der oder die Längsträger 8 können durch Verbindungsteile 6 an einem oder mehreren Dämpfungselementen 1, welche in diesem Fall eine axial ausreichende Länge aufweisen müssen, ersetzt sein. Die Längsachse 3 kann bei verschiedenen Anwendungen der erfindungsgemässen Vorrichtung 38 auch gekrümmt oder abgewinkelt sein.

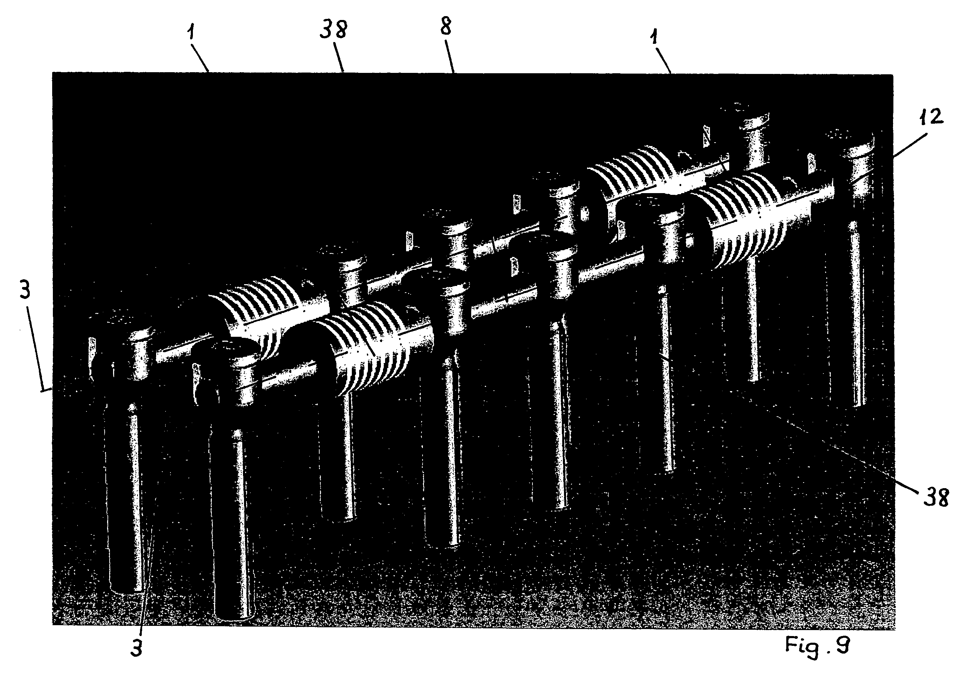

Fig. 9 zeigt eine Ausführungsform der erfindungsgemässen Vorrichtung, welche sich von der in Fig. 8 dargestellten Ausführungsform nur dadurch unterscheidet, dass sie zwei zu den Längsachsen 3 parallele Vorrichtungen 38 mit je zwei axial enständigen Dämpfungselementen 1 und je einem dazwischenliegend angeordneten Längsträger 8 umfasst. Ferner sind je fünf Pedikelschrauben 12 vorgesehen.

## Patentansprüche

1. Aggregat für eine Vorrichtung zur Stabilisierung benachbarter Wirbelkörper umfassend
A) ein Dämpfungselement (1), welches zwei zu einer Längsachse (3) koaxiale oder parallele Federelemente (2;4) und axial endständig ein erstes und ein zweites Verbindungsteil (5;6) umfasst; und
B) einen mit dem ersten Verbindungsteil (5) des Dämpfungselementes (1) verbindbaren rigiden Längsträger (8);
wobei
C) das Aggregat Befestigungselemente (7) umfasst, welche in Aufnahmemittel (13) von Pedikelschrauben (12) oder Pedikelhaken einführbar und lösbar mit Letzteren verbindbar sind;
D) das zweite Verbindungsteil (6) des Dämpfungselementes (1) stabförmig ausgebildet ist und als erstes Befestigungselement (7) dient;
E) ein zweites und ein drittes Befestigungselement (7) durch axiale Segmente des Längsträgers (8) gebildet werden; und
F) das erste Federlement (2) eine Federrate F und das zweite Federelement (4) eine Federrate f aufweist,
**dadurch gekennzeichnet, dass**
G) die Federraten F und f voneinander verschieden sind.

2. Aggregat nach Anspruch 1, **dadurch gekennzeichnet, dass** sich die beiden Federkonstanten F;f mindestens um den Faktor 1,2, vorzugsweise mindestens um den Faktor 5 unterscheiden.

3. Aggregat nach Anspruch 2, **dadurch gekennzeichnet, dass** sich die beiden Federkonstanten F;f um einen Faktor zwischen 10 und 100 unterscheiden.

4. Aggregat nach einem der Ansprüche 1 bis 3, **dadurch gekennzeichnet, dass** das Aggregat zusätzlich N Pedikelschrauben (12) oder Pedikelhaken umfasst, wobei N ≥ 3 ist; und
wobei
a) jede der N Pedikelschrauben (12) oder Pedikelhaken Aufnahmemittel (13) umfasst, welche die Aufnahme von longitudinalen Befestigungselementen (7) gestatten;
b) das Dämpfungselement (1) zwischen zwei benachbarten Pedikelschrauben (12) oder Pedikelhaken angeordnet ist; und
c) der Längsträger (8) zwischen einer der zwei benachbarten Pedikelschrauben (12) oder Pedikelhaken und einer weiteren Pedikelschraube (12) oder Pedikelhaken angeordnet ist.

5. Aggregat nach einem der Ansprüche 1 bis 3, **dadurch gekennzeichnet, dass** das Aggregat zusätzlich
i) eine mit einem ersten Wirbelkörper verbindbare erste Pedikelschraube (12) oder Pedikelhaken;
ii) eine mit einem zweiten Wirbelkörper verbindbare zweite Pedikelschraube (12) oder Pedikelhaken;
iii) eine mit einem dritten Wirbelkörper verbindbare dritte Pedikelschraube (12) oder Pedikelhaken umfasst;
wobei
iv) jede Pedikelschraube (12) oder Pedikelhaken Aufnahmemittel (13) umfasst, welche die Aufnahme von longitudinalen Befestigungselementen (7) gestatten; und
v) das Aggregat so ausgebildet ist, dass zwischen der ersten Pedikelschraube (12) oder Pedikelhaken und der zweiten Pedikelschraube (12) oder Pedikelhaken ein als Feder wirkendes Dämpfungselement (1) angeordnet ist; und
vi) zwischen der zweiten Pedikelschraube (12) oder Pedikelhaken und der dritten Pedikelschraube (12) oder Pedikelhaken der rigide Längsträger (8) angeordnet ist.

6. Aggregat nach einem der Ansprüche 1 bis 5, **dadurch gekennzeichnet, dass** die Federelemente (2;4) zur Längsachse (3) konzentrisch angeordnet sind.

7. Aggregat nach einem der Ansprüche 1 bis 6, **dadurch gekennzeichnet, dass**
a) das Aggregat ein zweites Dämpfungselement (1) umfasst;
b) das erste Ende des Längsträgers (8) mit dem ersten Verbindungsteil (5) des ersten Dämpfungselements (1) verbunden ist; und
c) das zweite Ende des Längsträgers (8) mit dem ersten Verbindungsteil (5) des zweiten Dämpfungselements (1) verbunden ist.

8. Aggregat nach Anspruch 7, **dadurch gekennzeichnet, dass** das zweite Verbindungsteil (6) des zweiten Dämpfungselements (1) als viertes Befestigungselement (7) dient.

9. Aggregat nach Anspruch 8, **dadurch gekennzeichnet, dass** N = 5 ist und das zweite, das dritte und ein fünftes Befestigungselement (7) durch axiale Segmente des Längsträgers (8) gebildet werden.

10. Aggregat nach einem der Ansprüche 1 bis 9, **dadurch gekennzeichnet, dass** die Verbindungsteile (5;6) mit den Federelementen (2;4) so zusammenfügbar sind, dass mindestens eines der Federelemente (2;4) mit den Verbindungsteilen (5;6) verbunden ist.

11. Aggregat nach einem der Ansprüche 1 bis 9, **dadurch gekennzeichnet, dass** mindestens eines der Verbindungsteile (5;6) mit mindestens einem Federelement (2;4) einstückig ist.

12. Aggregat nach einem der Ansprüche 1 bis 11, **dadurch gekennzeichnet, dass** mindestens ein Verbindungsteil (5) eine Büchse (30) mit einer zur Längsachse (3) koaxialen Zentralbohrung (32) umfasst und dass an der Büchse (30) Arretiermittel (31) zur Blockierung eines in die Zentralbohrung (32) einführbaren Stabes angeordnet sind.

13. Aggregat nach einem der Ansprüche 1 bis 12, **dadurch gekennzeichnet, dass** mindestens eines der Verbindungsteile (5;6) stabförmig ausgebildet ist.

14. Aggregat nach Anspruch 13, **dadurch gekennzeichnet, dass** die Zentralbohrung (32) in der Büchse (30) einen Durchmesser D aufweist und dass das zweite Verbindungsteil (6) kreiszylindrisch mit einem Durchmesser d ausgestaltet ist.

15. Aggregat nach Anspruch 14, **dadurch gekennzeichnet, dass** D ≅ d ist.

16. Aggregat nach einem der Ansprüche 12 bis 15, **dadurch gekennzeichnet, dass** die Arretiermittel (31) mindestens eine Schraube umfassen, welche quer zur Längsachse (3) in die Büchse (30) einschraubbar ist.

17. Aggregat nach einem der Ansprüche 1 bis 16, **dadurch gekennzeichnet, dass** mindestens eines der Federelemente (2;4) vorgespannt ist.

18. Aggregat nach einem der Ansprüche 1 bis 17, **dadurch gekennzeichnet, dass** die Federelemente (2;4) konstante Federraten F;f aufweisen.

19. Aggregat nach einem der Ansprüche 1 bis 18, **dadurch gekennzeichnet, dass** es einen zur Längsachse (3) orthogonalen, nierenförmigen Querschnitt aufweist.

20. Aggregat nach einem der Ansprüche 1 bis 19, **dadurch gekennzeichnet, dass** ein Federelement (4) als Druckfeder angeordnet ist.

21. Aggregat nach einem der Ansprüche 1 bis 20, **dadurch gekennzeichnet, dass** das zweite Federelement (4) im Innern des ersten Federelementes (2) angeordnet ist.

22. Aggregat nach Anspruch 21, **dadurch gekennzeichnet, dass** das zweite Federelement (4) eine zur Längsachse (3) koaxiale Zentralbohrung (33) umfasst.

23. Aggregat nach einem der Ansprüche 1 bis 22, **dadurch gekennzeichnet, dass** das erste Federelement (2) als Schraubenfeder ausgebildet ist und einen schraubenlinienförmigen Schlitz (34) aufweist.

24. Aggregat nach Anspruch 23, **dadurch gekennzeichnet, dass** das zweite Federelement (4) eine zum schraubenlinienförmigen Schlitz (34) am ersten Federelement (2) komplementäre, elastisch deformierbare Erhebung (35) umfasst, welche den Schlitz (34) verschliesst.

25. Aggregat nach einem der Ansprüche 1 bis 24, **dadurch gekennzeichnet, dass** ein Federelement (2;4) mit einer mehrgängigen Federwendel ausgestattet ist.

26. Aggregat nach einem der Ansprüche 20 bis 25, **dadurch gekennzeichnet, dass** das als Druckfeder angeordnete Federelement (4) aus einem Polymer, vorzugsweise aus Polycarbonaturethan (PCU) besteht.

27. Aggregat nach einem der Ansprüche 1 bis 25, **dadurch gekennzeichnet, dass** das zweite Federelement (4) mit einem der Verbindungsteile (5;6) einstückig ausgebildet ist.

28. Aggregat nach einem der Ansprüche 12 bis 27, **dadurch gekennzeichnet, dass** die Büchse (30) mit dem ersten Federelement (2) einstückig ist.

29. Aggregat nach einem der Ansprüche 1 bis 28, **dadurch gekennzeichnet, dass** des erste Federelement (2) mit beiden Verbindungsteilen (5;6) einstückig ist.

30. Aggregat nach einem der Ansprüche 1 bis 29, **dadurch gekennzeichnet, dass** die Federrate f des zweiten Federelementes (4) zwischen 50 N/mm und 5000 N/mm beträgt.

31. Aggregat nach Anspruch 30, **dadurch gekennzeichnet, dass** die Federrate f des zweiten Federelementes (4) zwischen 100 N/mm und 2000 N/mm beträgt.
